Europäisches Patentamt

European Patent Office

Office européen des brevets

(19)

(11) Publication number: **0 455 460 A2**

(12) **EUROPEAN PATENT APPLICATION**

(21) Application number: **91303891.5**

(22) Date of filing: **30.04.91**

(51) Int. Cl.⁵: **C12N 15/12, C12P 21/02, C12Q 1/68, C07K 15/00**

The microorganism(s) has (have) been deposited with American Type Culture Collection under number(s) ATCC 68309.

(30) Priority: **01.05.90 US 517288**

(43) Date of publication of application: **06.11.91 Bulletin 91/45**

(84) Designated Contracting States: **AT BE CH DE DK ES FR GB GR IT LI LU NL SE**

(71) Applicant: **E.R. SQUIBB & SONS, INC.**
**Lawrenceville-Princeton Road**
**Princeton New Jersey 08543-4000 (US)**

(72) Inventor: **Barbacid, Mariano**
**5 Stoney Creek Place**
**Lawrenceville, NJ (US)**
Inventor: **Klein, Rudiger**
**28 Linden Lane S.**
**Plainsboro, NJ (US)**

(74) Representative: **Crisp, David Norman et al**
**D. YOUNG & CO. 10 Staple Inn**
**London, WC1V 7RD (GB)**

(54) **Tyrosine kinase negative TRKB.**

(57)    Nucleic acid sequences, particularly DNA sequences, coding for all or part of a tyrosine kinase negative *trk*B protein, expression vectors containing the DNA sequences, host cells containing the expression vectors, and methods for detecting the DNA sequences or the corresponding RNA sequences. The invention also concerns polypeptide molecules comprising all or part of a *trk*B protein that lacks a catalytic tyrosine protein kinase domain (tyrosine kinase negative *trk*β protein).

EP 0 455 460 A2

# FIG. 1A

The existence of oncogenes has been known for some time. An oncogene may be broadly defined as a gene whose protein product, when present in certain host cells, can transform the cells to a cancerous phenotype. A proto-oncogene, on the other hand, may be broadly defined as a normal gene which can become "activated" to yield an oncogene. The first oncogenes discovered were the transforming genes of certain oncogenic viruses. Subsequently, it was discovered that oncogenes are also present in various eucaryotic cells. Included among these oncogenes is the oncogene designated as *trk*.

The *trk* locus was first identified in a human colon carcinoma where it became activated as an oncogene by a chromosomal rearrangement which fused its transmembrane and catalytic domains to a subset of sequences derived from a non-muscle tropomyosin gene. Martin-Zanca et al., Nature 319, 743-748 (1986). Additional *trk* oncogenes carrying activating sequences other than tropomyosin have been generated during the course of gene transfer assays. Kozma et al., EMBO J. 9, 147-154 (1988); Oskam et al., Proc. Natl. Acad. Sci. 9, 2964-2968 (1988). The *trk* proto-oncogene codes for a cell surface receptor with tyrosine protein kinase activity that is specifically expressed in the trigeminal and certain dorsal root ganglia.

A gene related to the *trk* proto-oncogene and designated *trk*B has recently been isolated from a mouse brain cDNA library. Klein, R. et al., EMBO J. 8, 3701-3709 (1989). The *trk*B proto-oncogene also codes for a cell surface receptor with tyrosine protein kinase activity. Mutated alleles (oncogenes) of both of these genes can trigger malignant transformation.

The present invention involves the surprising discovery that the *trk*B gene codes for a second cell surface receptor that lacks a catalytic tyrosine kinase domain. This protein has been designated "tyrosine kinase negative *trk*B receptor".

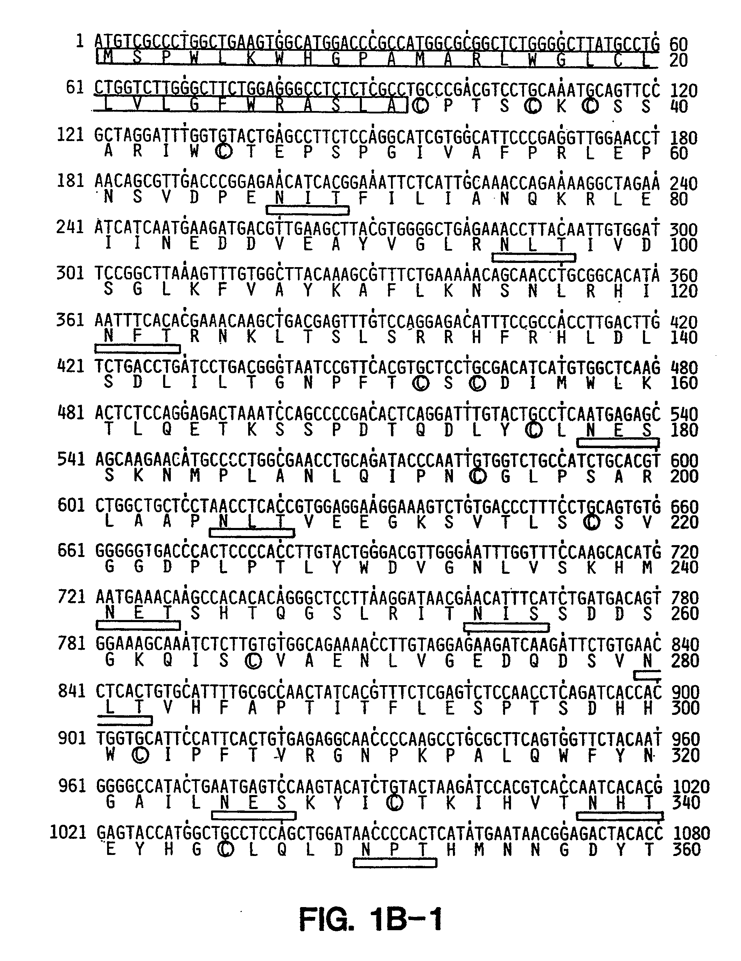

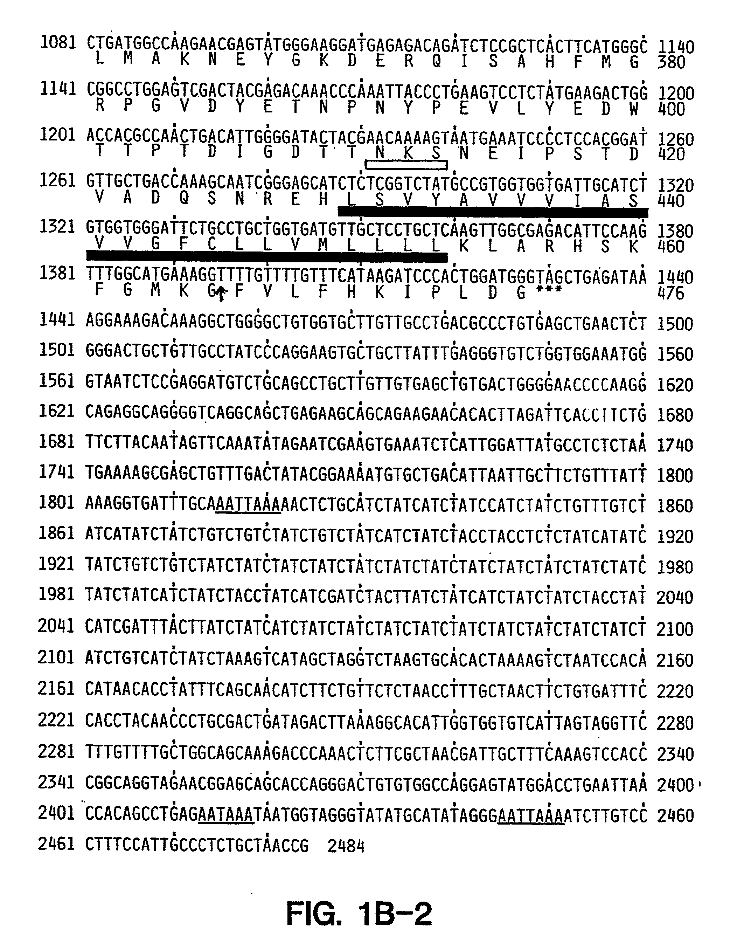

The present invention concerns an isolated nucleic acid molecule comprising a nucleic acid sequence coding for all or part of a tyrosine kinase negative *trk*B protein. Preferably, the nucleic acid molecule is a DNA (deoxyribonucleic acid) molecule, and the nucleic acid sequence is a DNA sequence. Further preferred is a DNA sequence having all or part of the nucleotide sequence substantially as shown in Figure 1B.

The present invention further concerns expression vectors containing all or part of a DNA sequence coding for a tyrosine kinase negative *trk*B protein.

The present invention additionally concerns prokaryotic or eukaryotic host cells transformed or transfected with an expression vector which contains all or part of a DNA sequence coding for a tyrosine kinase negative *trk*B protein.

The present invention also concerns methods for detecting nucleic acid sequences coding for all or a portion of a tyrosine kinase negative *trk*B protein.

The present invention further concerns polypeptide molecules comprising all or part of a tyrosine kinase negative *trk*B protein.

Various preferred features and embodiments of the present invention will now be described by way of non-limiting example with reference to the accompanying drawings, in which:

FIGURE 1 shows the nucleotide and deduced amino acid sequence analysis of pFRK42, a *trk*B tyrosine kinase negative cDNA clone.

FIGURE 2 shows the molecular analysis of *trk*B transcripts in adult mouse tissues.

FIGURE 3 shows the identification of *trk*B proteins expressed in NIH3T3 cells transfected with tyrosine kinase positive and tyrosine kinase negative *trk*B cDNA clones.

FIGURE 4 shows *trk*B expression in the hippocampal region of adult mouse brain.

FIGURE 5 shows *trk*B expression in the choroid plexus of adult mouse brain.

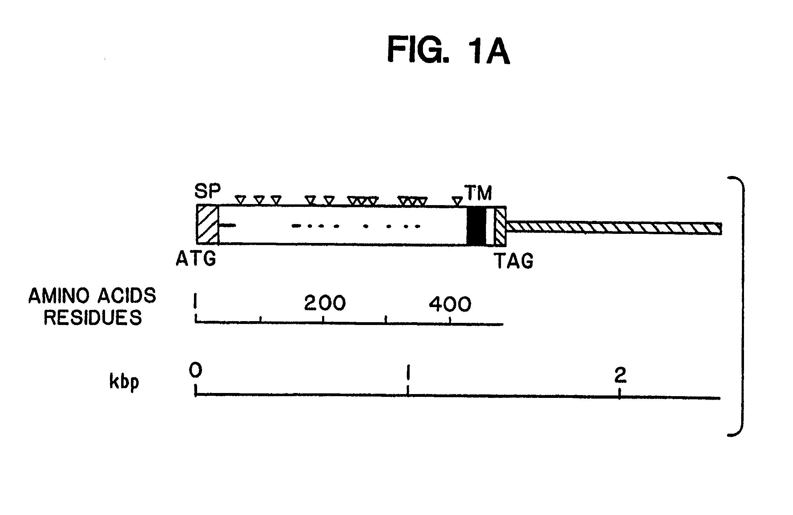

The present invention concerns an isolated nucleic acid molecule comprising a nucleic acid sequence coding for all or part of a tyrosine kinase negative *trk*B protein. Preferably, the nucleic acid molecule is a DNA molecule and the nucleic acid sequence is a DNA sequence. Further preferred is a DNA sequence having all or part of the nucleotide sequence substantially as shown in Figure 1B, or a DNA sequence complementary to this DNA sequence.

As used in this application, the term "tyrosine kinase negative *trk*B protein" means a *trk*B protein lacking a tyrosine protein kinase catalytic domain.

The DNA sequences of the present invention may be isolated from a variety of sources, although the presently preferred sequence has been isolated from a mouse cDNA (complementary DNA) library. The exact amino acid sequence of the polypeptide molecule produced will vary with the initial DNA sequence.

The DNA sequences of the present invention may be obtained using various methods well-known to those of ordinary skill in the art. At least three alternative principal methods may be employed:

(1) the isolation of a double-stranded DNA sequence from genomic DNA which contains the sequence;

(2) the chemical synthesis of the DNA sequence; and

(3) the in vitro synthesis of a double-stranded DNA sequence by enzymatic reverse transcription of mRNA (messenger RNA) encoded by the DNA sequence followed by isolation of the DNA sequence.

The last-mentioned method involves formation of a DNA complementary to the mRNA, and is generally referred to as a cDNA method. In order to isolate a cDNA sequence coding for all or part of a tyrosine kinase negative *trk*B protein, a plasmid-borne cDNA library is prepared by reverse transcription of mRNA molecules, some of which are encoded by the DNA sequence coding for all or part of a tyrosine kinase negative *trk*B protein.

These cDNA libraries may be prepared using various methods known in the art. For example, a double stranded cDNA copy of the mRNA is first generated using reverse transcriptase. After the formation of "sticky" ends (e.g., by digestion with a restriction endonuclease such as Eco RI), the double stranded cDNA with sticky ends is ligated to about a 2-fold molar excess of phage vector DNA, for example λgt10 or λgt11 DNA, which has also been digested with a restriction endo-nuclease such as Eco RI to yield sticky ends, and phosphatased to prevent ligation without a cDNA insert. The ligation mixture is then packaged into infectious phage particles in vitro, and transformed into host bacteria. For λgt10 based libraries, a suitable host bacteria is *Escherichia coli C600hf1A*, while for λgt11 based libraries, a suitable host bacteria is *Escherichia coli* Y1088.

Once a cDNA library has been created, it must be screened to identify bacteriophage plaques or bacterial colonies containing the DNA sequence coding for all or part of a tyrosine kinase negative *trk*B protein.

For example, labeled single stranded DNA probe sequences duplicating a sequence present in the target cDNA coding for all or part of a tyrosine kinase negative *trk*B protein may be employed in DNA/DNA hybridization procedures carried out on cloned copies of the cDNA which have been denatured to single stranded form. If the cDNA library is to be screened in this manner, virtually any cloning vector, for example, λgt10, may be employed.

A cDNA library may also be screened for a cDNA coding for all or part of a tyrosine kinase negative *trk*B protein using immunoblotting techniques. If the library is to be screened in this manner, an appropriate *Escherichia coli* expression vector should be used. In one such approach, appropriate vectors are based on expression of a fusion protein in which a segment of the peptide of interest (i.e., all or part of a tyrosine kinase negative *trk*B protein) is fused to a highly expressed, stable *Escherichia coli* protein. For example, λgt11, which is an expression vector in which the cloned sequences coding for all or part of a tyrosine kinase negative *trk*B protein may be fused to coding sequences for B-galactosidase, may be employed.

In one typical screening method suitable for either immunoblotting or hybridization techniques, the cDNA library is first spread out on agarose plates, and then the clones are transferred to filter membranes, for example, nitrocellulose membranes. A DNA probe may then be hybridized or an antibody may then be bound to the clones to identify those clones containing the cDNA coding for all or part of a tyrosine kinase negative *trk*B protein.

The DNA sequences of the present invention may be used in a variety of ways in accordance with the present invention. For example, they may be used as DNA probes to screen other cDNA or genomic DNA libraries to select by hybridization other DNA sequences that code for proteins related to tyrosine kinase negative *trk*B.

The DNA sequences of the present invention may also be used to prepare various mutations. Such mutations may be either degenerate, i.e., the mutation does not change the amino acid sequence encoded by the mutated codon, or non-degenerate, i.e., the mutation changes the amino acid sequence encoded by the mutated codon. Both types of mutations may be advantageous in producing or using the polypeptides of the present invention. For example, these mutations may permit higher levels of production, easier purification, or provide additional restriction endonuclease recognition sites. Such mutations are included within the scope of the present invention.

The present invention further concerns expression vectors containing a DNA sequence coding for all or part of a tyrosine kinase negative *trk*B protein. The expression vectors preferably contain all or part of the DNA sequence having the nucleotide sequence substantially as shown in Figure 1B. Further preferred are expression vectors comprising one or more control DNA sequences operatively linked to the DNA sequence coding for all or part of a tyrosine kinase negative *trk*B protein. As used in this context, the term "operatively linked" means that the control DNA sequences are capable of directing the replication and/or the expression of the DNA sequence coding for all or part of a tyrosine kinase negative *trk*B protein.

Expression vectors of utility in the present invention are often in the form of "plasmids", which refer to circular double stranded DNA loops which, in their vector form, are not bound to the chromosome. However, the invention is intended to include such other forms of expression vectors which serve equivalent functions and which become known in the art subsequently hereto.

Expression vectors useful in the present invention typically contain an origin of replication, a promoter located in front of (i.e., upstream of) the DNA sequence and followed by the DNA sequence coding for all or part of a tyrosine kinase negative *trk*B protein, replication termination sequences and the remaining vector. The expression vectors may also include other DNA sequences known in the art, for example, stability leader sequences which provide for stability of the expression product, secretory leader sequences which provide for secretion of the expression product, regulatory sequences which allow expression of the structural gene to be modulated (e.g., by the presence or absence of nutrients in the growth medium), marking sequences which are capable of providing phenotypic selection in transformed host cells, and sequences which provide sites for cleavage by restriction endonucleases. The characteristics of the actual expression vector used must be compatible with the host cell which is to be employed. For example, when cloning in a mammalian cell system, the expression vector should contain promoters isolated from the genome of mammalian cells, (e.g., mouse metallothionien promoter), or from viruses that grow in these cells (e.g., vaccinia virus 7.5 K promoter). An expression vector as contemplated by the present invention is at least capable of directing the replication, and preferably the expression, of the DNA sequences of the present invention. Suitable origins of replication include, for example, those of the E. coli vector pBR322. Suitable promoters include, for example, the long terminal repeat of avian (e.g., Rous sarcoma virus) or mammalian (e.g., Moloney sarcoma virus) retroviruses, the early region of the DNA virus SV40 and the promoters of certain cellular genes such as metallothionine and thymidine kinase. Suitable termination sequences include, for example, polyadenylation sequences such as those of the DNA virus SV40. As selectable markers, the bacterial neo and gpt genes are most suitable. In general, vectors derived from pBR322, for example, pUC18 and pUC19, may be employed. All of these materials are known in the art and are commercially available.

Particularly preferred is the expression vector designated pFRK42, described herein below, which contains the DNA sequence coding for a tyrosine kinase negative trkB protein, or expression vectors with the identifying characteristics of pFRK42.

A host cell (Escherichia coli strain DH5) containing pFRK42 was deposited with the American Type Culture Collection, Rockville, Maryland on April 18, 1990 under the Budapest Treaty and assigned ATCC accession no. 68309. The resulting transformed host cell has been designated DH5(pFRK42).

Suitable expression vectors containing the desired coding and control sequences may be constructed using standard recombinant DNA techniques known in the art, many of which are described in Maniatis, T. et al., Molecular Cloning: A Laboratory Manual, Cold Spring Harbor Laboratory, Cold Spring Harbor, NY (1982). Isolated plasmids or DNA fragments may be cleaved, tailored and religated to form the desired expression vector.

Cleavage of plasmids or DNA fragments, may be performed, for example, by treating with one or more restriction enzymes under suitable reaction conditions. Suitable restriction enzymes are commercially available and include, for example, Bal I, Eco RI, Bam HI and Xho 1. Suitable reaction conditions such as appropriate buffers, substrate amounts and reaction times and temperatures for a particular restriction enzyme are known in the art, and are generally specified by the manufacturer of the enzyme. In general, about 1 µg of a plasmid or DNA fragment is used with about 1 unit of enzyme in about 20 µl of buffer solution. Incubation times of about 1 hour at 37°C are typical.

Tailoring of plasmids or DNA fragments may be performed by various methods known in the art. For example, if DNA molecules with blunt ends are required, the Klenow fragment of DNA Polymerase I may be employed. This enzyme is commercially available, and suitable reaction conditions are known in the art and are usually specified by the manufacturer. Treatment of a DNA preparation with about 10 units of Klenow fragment for about 15 minutes at about 15°C is typical.

Ligation of plasmids or DNA fragments may also be performed by various methods known in the art. For example, DNA ligase from bacteriophage $T_4$ may be employed. This enzyme is commercially available, and suitable reaction conditions are known in the art and are usually specified by the manufacturer. Typically, equimolar amounts of the desired DNA molecules are treated with about 10 units of $T_4$ DNA ligase per 0.5 µg of DNA.

The present invention additionally concerns host cells containing an expression vector which contains a DNA sequence coding for all or part of a tyrosine kinase negative trkB protein. The host cells preferably contain an expression vector which contains all or part of the DNA sequence having the nucleotide sequence substantially as shown in Figure 1. Further preferred are host cells containing an expression vector comprising one or more control DNA sequences capable of directing the replication and/or the expression of and operatively linked to a DNA sequence coding for all or part of a tyrosine kinase negative trkB protein. Suitable host cells include both prokaryotic and eukaryotic cells. Suitable prokaryotic host cells include, for example, Escherichia coli cells. Suitable eukaryotic host cells include, for example, mouse BALB 3T3 and NIH3T3 cells, rat RAT-2 and RAT-4 cells, hamster CHO cells, and human HeLa and HOS cells.

Particularly preferred as host cells are mouse cells such as NIH3T3 cells.

Expression vectors may be introduced into host cells by various methods known in the art. For example, transfection of host cells with expression vectors may be carried out by the calcium phosphate precipitation method. However, other methods for introducing expression vectors into host cells, for example, electroporation, nuclear injection or protoplast fusion, may also be employed.

Once an expression vector has been introduced into an appropriate host cell, the host cell may be cultured under conditions permitting expression of large amounts of the desired polypeptide, in this case a polypeptide molecule comprising all or part of a tyrosine kinase negative *trk*B protein. Such polypeptides are useful in the study of the characteristics of the tyrosine kinase negative *trk*B protein, for example, its role in malignant transformation.

Host cells containing an expression vector which contains a DNA sequence coding for all or part of a tyrosine kinase negative *trk*B protein may be identified by one or more of the following four general approaches: (a) DNA-DNA hybridization; (b) the presence or absence of marker gene functions; (c) assessing the level of transcription as measured by the production of tyrosine kinase negative *trk*B mRNA transcripts in the host cell; and (d) detection of the gene product immunologically.

In the first approach, the presence of a DNA sequence coding for all or part of a tyrosine kinase negative *trk*B protein can be detected by DNA-DNA hybridization using probes complementary to the DNA sequence.

In the second approach, the recombinant expression vector host system can be identified and selected based upon the presence or absence of certain marker gene functions (e.g., thymidine kinase activity, resistance to antibiotics, etc.). A marker gene can be placed in the same plasmid as the DNA sequence coding for all or part of a tyrosine kinase negative *trk*B protein under the control of the same or a different promoter used to control the tyrosine kinase negative *trk*B coding sequence. Expression of the marker gene in response to induction or selection indicates expression of the DNA sequence coding for all or part of a tyrosine kinase negative *trk*B protein.

In the third approach, the production of tyrosine kinase negative *trk*B mRNA transcripts can be assessed by hybridization assays. For example, polyadenylated RNA can be isolated and analyzed by Northern blotting using a probe complementary to the RNA sequence. Alternatively, the total nucleic acids of the host cell may be extracted and assayed for hybridization to such probes.

In the fourth approach, the expression of all or part of a tyrosine kinase negative *trk*B protein can be assessed immunologically, for example, by Western blotting.

The DNA sequences of expression vectors, plasmids or DNA molecules of the present invention may be determined by various methods known in the art. For example, the dideoxy chain termination method as described in Sanger et al., Proc. Natl. Acad. Sci. USA 74, 5463-5467 (1977), or the Maxam-Gilbert method as described in Proc. Natl. Acad. Sci. USA-74, 560-564 (1977) may be employed.

It should, of course, be understood that not all expression vectors and DNA control sequences will function equally well to express the DNA sequences of the present invention. Neither will all host cells function equally well with the same expression system. However, one of ordinary skill in the art may make a selection among expression vectors, DNA control sequences, and host cells without undue experimentation and without departing from the scope of the present invention.

The present invention further concerns a method for detecting a nucleic acid sequence coding for all or part of a tyrosine kinase negative *trk*B protein comprising contacting the nucleic acid sequence with a detectable marker which binds specifically to at least a portion of the nucleic acid sequence, and detecting the marker so bound. The presence of bound marker indicates the presence of the nucleic acid sequence. Preferably, the nucleic acid sequence is a DNA sequence having all or part of the nucleotide sequence substantially as shown in Figure 1B. Also preferred is a method in which the DNA sequence is a genomic DNA sequence. A DNA sample containing the DNA sequence may be isolated using various methods for DNA isolation which are well-known to those of ordinary skill in the art. For example, a genomic DNA sample may be isolated from tissue by rapidly freezing the tissue from which the DNA is to be isolated, crushing the tissue to produce readily digestible pieces, placing the crushed tissue in a solution of proteinase K and sodium dodecyl sulfate, and incubating the resulting solution until most of the cellular protein is degraded. The digest is then deprotenized by successive phenol/chloroform/ isoamyl alcohol extractions, recovered by ethanol precipitation, and dried and resuspended in buffer.

Also preferred is the method in which the nucleic acid sequence is an RNA sequence. Preferably, the RNA sequence is an mRNA sequence. Additionally preferred is the method in which the RNA sequence is located in the cells of a tissue sample. An RNA sample containing the RNA sequence may be isolated using various methods for RNA isolation which are well-known to those of ordinary skill in the art. For example, an RNA sample may be isolated from cultured cells by washing the cells free of media and then lysing the cells by placing them in a 4 M guanidinium solution. The viscosity of the resulting solution is reduced by drawing the lysate through a 20 gauge needle. The RNA is then pelleted through a $CsCl_2$ step gradient, and the supernatant fluid from the gradient carefully removed to allow complete separation of the RNA, found in the pellet, from contaminating DNA and protein.

The detectable marker useful for detecting a nucleic acid sequence coding for all or part of a tyrosine kinase negative *trk*B protein, may be a labeled DNA sequence, including a labeled cDNA sequence, having a nucleotide sequence complementary to at least a portion of the DNA sequence coding for all or part of a tyrosine kinase negative *trk*B protein.

6

The detectable marker may also be a labeled sense or antisense RNA sequence having a nucleotide sequence complementary to at least a portion of the DNA sequence coding for all or part of a tyrosine kinase negative trkB protein.

The detectable markers of the present invention may be labeled with commonly employed radioactive labels, such as $^{32}$P and $^{35}$S, although other labels such as biotin may be employed. Various methods well-known to those of ordinary skill in the art may be used to label the detectable markers. For example, DNA sequences and RNA sequences may be labeled with $^{32}$P or $^{35}$S using the random primer method.

Once a suitable detectable marker has been obtained, various methods well-known to those of ordinary skill in the art may be employed for contacting the detectable marker with the sample of interest. For example, DNA-DNA, RNA-RNA and DNA-RNA hybridizations may be performed using standard procedures known in the art. In a typical DNA-DNA hybridization procedure for detecting DNA sequences coding for all or part of a tyrosine kinase negative trkB protein in genomic DNA, the genomic DNA is first isolated using known methods, and then digested with one or more restriction enzymes. The resulting DNA fragments are separated on agarose gels and denatured in situ. After prehybridization to reduce nonspecific hybridization, a radiolabeled nucleic acid probe is hybridized to the immobilized DNA fragments. The filter is then washed to remove unbound or weakly bound probe, and is then autoradiographed to identify the DNA fragments that have hybridized with the probe.

The presence of bound detectable marker may be detected using various methods well-known to those of ordinary skill in the art. For example, if the detectable marker is radioactively labeled, autoradiography may be employed. Depending on the label employed, other detection methods such as spectrophotometry may also be used.

The present invention further concerns polypeptide molecules comprising all or part of a tyrosine kinase negative trkB protein, said polypeptide molecules preferably having all or part of the amino acid sequence substantially as shown in Figure 1B. Further preferred are polypeptide molecules having amino acid sequences which are at least 90% homologous to all or part of the amino acid sequence substantially as shown in Figure 1B. Also preferred are polypeptide molecules comprising all or part of a tyrosine kinase negative trkB protein which are glycosylated.

All amino acid residues identified herein are in the natural L-configuration. In keeping with standard polypeptide nomenclature, J. Biol. Chem. 243, 3557-3559 (1969), abbreviations for amino acid residues are as shown in the following Table of Correspondence:

## TABLE OF CORRESPONDENCE

| SYMBOL | | AMINO ACID |
|---|---|---|
| 1-Letter | 3-Letter | |
| Y | Tyr | L-tyrosine |
| G | Gly | L-glycine |
| F | Phe | L-phenylalanine |
| M | Met | L-methionine |
| A | Ala | L-alanine |
| S | Ser | L-serine |
| I | Ile | L-isoleucine |
| L | Leu | L-leucine |
| T | Thr | L-threonine |
| V | Val | L-valine |
| P | Pro | L-proline |
| K | Lys | L-lysine |
| H | His | L-histidine |
| Q | Gln | L-glutamine |
| E | Glu | L-glutamic acid |
| W | Trp | L-tryptophan |
| R | Arg | L-arginine |
| D | Asp | L-aspartic acid |
| N | Asn | L-asparagine |
| C | Cys | L-cysteine |

All amino acid sequences are represented herein by formulas whose left to right orientation is in the conventional direction of amino-terminus to carboxy-terminus.

The polypeptides of the present invention may be obtained by synthetic means, i.e., chemical synthesis of the polypeptide from its component amino acids, by methods known to those of ordinary skill in the art. For example, the solid phase procedure described by Houghton et al., Proc. Natl. Acad. Sci. 82, 5135 (1985) may be employed. It is preferred that the polypeptides be obtained by production in prokaryotic or eukaryotic host cells expressing a DNA sequence coding for all or part of a tyrosine kinase negative trkB protein, or by in vitro translation of the nMRA encoded by a DNA sequence coding for all or part of a tyrosine kinase negative trkB protein. For example, the DNA sequence of Figure 1 may be chemically synthesized and inserted into a suitable expression vector, which in turn may be used to transform a suitable host cell. The recombinant host cell may then be cultured to produce the tyrosine kinase negative trkB protein. Techniques for the production of polypeptides by these means are known in the art, and are described herein.

The polypeptides produced in this manner may then be isolated and purified to some degree using various protein purification techniques. For example, chromatographic procedures such as ion exchange chromatography, gel filtration chromatography and immunoaffinity chromatography may be employed.

The polypeptides of the present invention may be used in a wide variety of ways. For example, the polypeptides may be used to prepare in a known manner polyclonal or monoclonal antibodies capable of binding the polypeptides. These antibodies may in turn be used for the detection of the polypeptides of the present invention in a sample, for example, a cell or tissue sample, using immunoassay techniques, for example, radio-immunoassay or enzyme immunoassay. The antibodies may also be used in affinity chromatography for purifying the polypeptides of the present invention and isolating them from various sources.

The polypeptides of the present invention have been defined by means of determined DNA and deduced amino acid sequencing. Due to the degeneracy of the genetic code, other DNA sequences which encode the same amino acid sequence as depicted in Figure 1B may be used for the production of the polypeptides of the present invention. In addition, it will be understood that allelic variations of these DNA and amino acid sequences naturally exist, or may be intentionally introduced using methods known in the art. These variations may be demonstrated by one or more amino acid differences in the overall sequence, or by deletions, substitutions, insertions, inversions or additions of one or more amino acids in said sequence. Such amino acid substitutions may be made, for example, on the basis of similarity in polarity, charge, solubility, hydrophobicity, hydrophilicity and/or the amphiphathic nature of the residues involved. For example, negatively charged amino acids include aspartic acid and glutamic acid; positively charged amino acids include lysine and arginine; amino acids with uncharged polar head groups or nonpolar head groups having similar hydrophilicity values include the following: leucine, isoleucine, valine; glycine, alanine; asparagine, glutamine; serine, threonine; phenylalanine, tyrosine. Other contemplated variations include salts and esters of the aforementioned polypeptides, as well as precursors of the aforementioned polypeptides, for example, precursors having N-terminal substituents such as methionine, N-formylmethionine and leader sequences. All such variations are included within the scope of the present invention.

The following examples are further illustrative of the present invention. These examples are not intended to limit the scope of the present invention, and provide further understanding of the invention.

## Example 1

### Isolation of Tyrosine Kinase Negative *trkB* cDNA Clones

Tyrosine kinase negative *trk*B cDNA clones were isolated by hybridizing an adult mouse brain cDNA library (Citri, Y. et al., Nature 326, 42-47 (1987)) with a 207 bp DNA fragment purified by gel electrophoresis from a tyrosine protein kinase positive *trk*B cDNA clone which corresponded to a portion of the extracellular domain of the *trk*B gene [nucleotides 1181-1387 of pFRK43, the *trk*B cDNA clone described in Klein, R. et al., EMBO J. 8, 3701-3709 (1989)]. Hybridizations were performed according to the method described in Klein, R. et al., EMBO J. 8, 3701-3709 (1989). Nucleotide sequence analysis of the 5' and 3' ends of eight recombinant phages revealed that the 3' ends of four of them contained sequences unrelated to those of pFRK43. The longest of these new cDNA clones (2.5 kbp) was subcloned in pBluescript (Stratagene, La Jolla, CA), according to standard methods described in Maniatis, et al., supra. Briefly, 600 ng of the recombinant phage DNA was digested with 60 units of the restriction endonuclease Eco RI (Boehringer Mannheim, Indianapolis, IN) at 37°C for 2 hours. A 2.5 kb Eco RI cDNA fragment was separated from phage DNA by electrophoresis through a 1% agarose gel containing 0.5 μg/ml ethidium bromide. An agarose block containing the 2.5 kb Eco RI cDNA fragment was excised from the gel, and the cDNA electroeluted from the agarose by electrophoresis within dialysis tubing. The cDNA fragment was pipetted from the dialysis tubing, cleaned by passage through an "elutip" column (Schleicher & Schuell, Keene NH), ethanol precipitated and resuspended in Tris/EDTA buffer. Several ligation reactions were set up by incubating various molar ratios of cDNA fragment and EcoRI-linearized pBluescript KS M13- plasmid DNA (Stratagene) in the presence of T4 DNA ligase (Bethesda Research Labs, Gaithersburg, MD) at 15°C for 20 hours. The resulting plasmid, designated pFRK42, was then used to transform competent *Escherchia coli* (strain DH5) cells according to the procedure described in Cohen, S.N. et al., Proc. Natl. Acad. Sci. USA 69, 2110 (1972), which were subsequently plated onto ampicillin - coated agar plates. "Miniprep" DNA was prepared from recombinant DH5 cells and subjected to Eco RI restriction analysis. Positive DH5 cells containing pFRK42 as visualized by the presence of the 2.5 kb cDNA fragment after agarose electrophoresis were grown up in 1 liter liquid cultures in order to prepare large-scale plasmid DNA. pFRK42 was submitted to complete nucleotide sequence analysis by the dideoxy chain termination method (See, Sanger, F. et al., supra) using double stranded DNA, synthetic oligonucleotides and modified T7 DNA polymerase (Sequenase, United States Biochemicals, Cleveland, OH).

As shown in FIGURE 1, this cDNA clone consists of 2489 bp of which nucleotides 1 to 1394 are identical to those present in pFRK43. These sequences correspond to those coding for the putative signal peptide (nucleotides 1 to 93), extracellular domain (nucleotides 94 to 1287), transmembrane region (nucleotides 1288-1359) and the first twelve amino acid residuals of the cytoplasmic domain of gp145 $trkB$ (the glycosylated form of protein encoded by the pFRK43 tyrosine kinase positive cDNA clone).

The homology between the pFRK42 and pFRK43 cDNA clones abruptly ends at nucleotide 1395 (nucleotide 1906 in pFRK43). The unique sequence of pFRK42 exhibits an in-frame terminator codon (TAG) just 33 nucleotides downstream from the point of divergence with pFRK43. The remaining nucleotides (positions 1432 to 2484) represent 3' non-coding sequences. These sequences include three putative polyadenylation signals (nucleotides 1815-1821, 2413-2418 and 2445-2451) and a streak of around 200 bp in which the basic TCTATCTA sequence appears as a highly repeated motif. These results indicate that pFRK42 can only code for a 476 amino acid-long polypeptide with a molecular mass of 53,185 daltons. Residues 1 to 465 of this putative polypeptide are identical to those of gp145 *trkB*. These residues encompass the entire extracellular and transmembrane domains. However, the product of pFRK42 has a short cytoplasmic domain of 23 residues of which the last eleven are unique to this molecule. Therefore, the predicted product of pFRK42 lacks the tyrosine protein kinase catalytic domain present in gp145 *trkB*.

Example 2

Northern Blot Analysis of trkB Transcripts in Adult Mouse Tissue

To determine whether the tyrosine kinase negative pFRK42 cDNA clone represents a faithful copy of a *trkB* transcript, poly A-containing RNA was isolated from brain, liver and lung tissue and submitted to Northern blot analysis using as probes short (250 bp-370 bp) DNA fragments corresponding to specific regions of this clone.

RNA isolation and northern blot analysis were done as described in Maniatis et al., supra. Probes were generated by amplification of pFRK42 or pFRK43 cDNA sequences using the polymerase chain reaction (PCR) method. Each PCR reaction mixture (100 μl) contained template DNA, 200 μM each of ATP, CTP, GTP and TTP, sense and antisense amplimers and Taq DNA polymerase (2.5 units) in 50 mM KCl, 10 mM Tris pH 8·3, 1·5 mM MgCl$_2$ and 0·1% gelatin (w/v). For Probe A, the template used was pFRK43 DNA (2 ng), the sense amplimer used was #P010-1 (3 μM) having the sequence 5′-CCGCTAGGATCCGGTGTACTGAGCCTTCTC-3′, and the antisense amplimer used was #P011-1 (3 μM) having the sequence 5′-CCTGGAGGATCCTGAGCCACATGATG- TCGC-3′. For Probe E, the template used was pFRK42 DNA (2 mg), the sense amplimer used was #P004-0 (3 μM) having the sequence 5′-GCTGGATCCGTGGTGCTTGT-TGCCTG-3′, and the antisense amplimer used was #P005-1 (3 μM) having the sequence 5′-TCACTTGGATCC-TATATTTGAACTATTGTA-3′. For Probe K, the template used was pFRK42 DNA (2 ng), the amplimer used was #P033-0 (3 μM) having the sequence 5′-GTCATAGCTAAGCTTAAGTGCACACTAAAAGTC-3′ and the antisense amplimer used was #P034-0 (3 μM) having the sequence 5′-GGACAGGATCCTAATTCCCTATATGCATATAC-3′. For Probe D, the template used was pFRK43 DNA (5 ng), the sense amplimer used was #P003-1 (4.5 μM) having the sequence 5′-CTGGGATCCGCTATCGAACAATGAGG-3′ and the antisense amplimer used was #P002-0 having the sequence 5′-AAGGGATCCGTCGTGTAGGCCAGTCTG-3′. For Probe H, the template used was pFRK43 DNA (2 ng), the sense amplimer used was #P014-2 (3 μM) having the sequence 5′-CACATTGGATCCGCACATCAAGAGACACAA-3′, and the antisense amplimer was #P015-1 (3 μM) having the sequence 5′-GGTCGGGATCCACACAGACACCGTAGAACT-3′. For Probe G, the template used was pFRK43 DNA (1 ng), the sense amplimer used was #P008-1 (3 μM) having the sequence 5′-TGAGGATCCCACCGATATCGATATTCGTGCC-3′, and the antisense amplimer used was # P009-0 (3 μM) having the sequence 5′-GCTGCCGGATCCAGTCAGTCGGAGTGCGTG-3.

The polymerase chain reaction was then performed using the following thermal cycling program:
Segment 1 - heat samples to 94°C within no less than 30 seconds;
Segment 2 - incubate samples at 94°C for 1 minute;
Segment 3 - cool samples to 45°C in no less than 1 minute;
Segment 4 - incubate samples at 45°C for 2 minutes;
Segment 5 - heat samples to 72°C in no less than 30 seconds;
Segment 6 - incubate samples at 72°C for 5 minutes.

This thermal cycle consisting of segments 1-6 was repeated 25 times with an automatic 10 second extension of segment 6 after each cycle. After finishing with all 25 cycles, the samples were incubated an additional 7 minutes at 72°C and then stored at 4°C for further analysis. Amplified DNAs were analyzed by agarose gel electrophoresis, gel purified, phenol/chloroform extracted and ethanol precipitated according to standard molecular biology procedures (Maniatis et al., supra). 100 ng of each DNA was labeled using a "NICK" translation kit (Amersham, Arlington Heights, IL) in the presence of$^{32}$P-labeled αdCTP (Dupont, Wilmington, DE). Unincorporated nucleotides were separated from the probes by affinity chromatography through "elutip" columns (Schleicher & Schuell). Hybridization was performed under high-stringency conditions (42°C in 5 x SSC [SSC = 35.06 g/l NaCl, 17.65 g/l Na-Citrate, pH 7.0], 50% formamide, 1 x Denhardt's solution and 10% dextran sulphate) for 48 hours. Hybridized filters were washed at 60°C for 30 minutes in 2 x SSC, 0.1% sodium dodecyl sulphate (SDS) (3 times) and in 0.1 x SSC, 0.1% SDS (1 time) and exposed to Kodak X-OMAT film for 1-4 days with the help of intensifier screens. The amounts of RNA loaded in each lane were controlled by hybridization to a [$^{32}$P]-labeled β-actin probe.

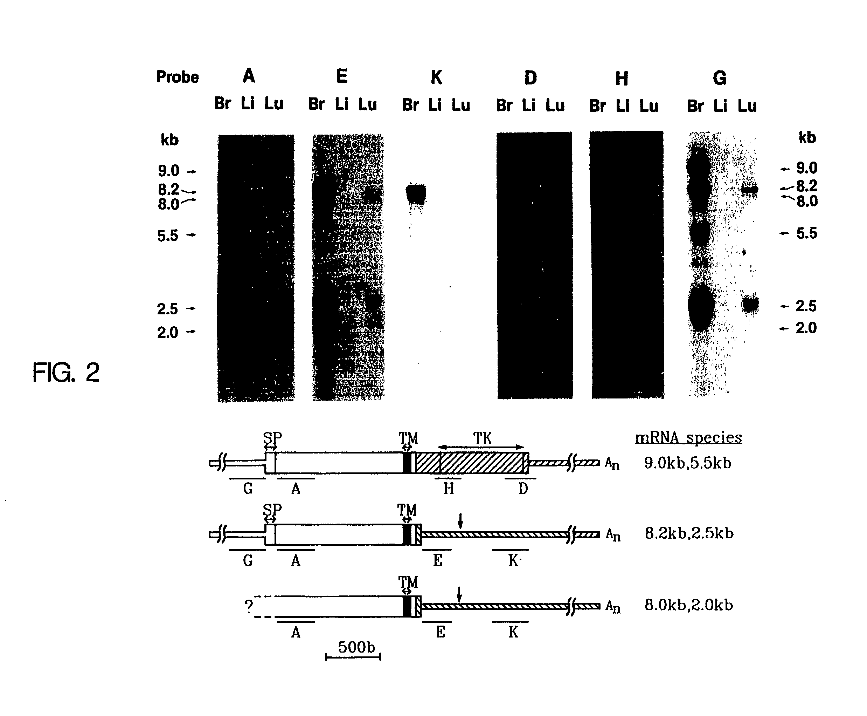

As shown in FIGURE 2, probe A, a DNA fragment (nucleotides 119-490) corresponding to those sequences coding for the amino terminus of the extracellular domain of the *trk*B product, recognized at least six distinct brain mRNAs of 9.0 kb, 8.2 kb, 8.0 kb, 5.5 kb, 2.5 kb and 2.0 kb. Four of these transcripts, including those of 8.2 kb, 8.0 kb, 2.5 kb and 2.0 kb, were also found in lung, albeit at lower levels. No *trk*B expression was observed in liver tissue.

Each of the above transcripts have been previously identified with a pFRK43-derived cDNA probe that contained most of the sequences coding for gp145$^{trkB}$. In contrast, when the same RNA blots were hybridized with probe E, a DNA fragment derived from sequences specific to the tyrosine kinase negative pFRK42 cDNA (nucleotides 1454-1711), only the 8.2 kb, 8.0 kb, 2.5 kb and 2.0 kb *trk*B transcripts could be identified. A second DNA fragment (probe K) derived from pFRK42-specific sequences located downstream from the repeated motif region (nucleotides 2119-2460), only recognized the long 8.2 kb and 8.0 kb transcripts. These observations suggest that the pFRK42 cDNA clone was derived from one of these two transcripts and that the AATTAAA sequence located at positions 1815 to 1821 (FIGURE 1) may serve as the polyadenylation signal utilized to generate the short tyrosine kinase negative 2.5 kb and 2.0 kb transcripts.

To determine which of the *trk*B transcripts contain sequences coding for the tyrosine protein kinase catalytic domain (tyrosine kinase positive transcripts), two additional probes designated H (nucleotides 2089-2343 in pFRK43) and D (nucleotides 2767-3057 in pFRK43) were prepared by PCR as described above. As shown in FIGURE 2, both of these pFRK43-specific probes efficiently recognized the 9.0 kb and 5.5 kb transcripts, the two mRNA species that did not hybridize to the pFRK42-specific E probe. Both of these tyrosine kinase-specific probes (H and D) failed to recognize the four tyrosine kinase negative transcripts that hybridized to pFRK42-derived probes E and K (FIGURE 2). These results demonstrate that the *trk*B locus can code for two classes of transcripts, one of which codes for gp145$^{trkB}$, a classical tyrosine protein kinase cell surface receptor, and another one that may direct the synthesis of a related molecule that lacks most of the cytoplasmic region including the tyrosine kinase catalytic domain.

Northern blot analysis of these *trk*B mRNAs with a probe derived from the 5′ non-coding sequences present in pFRK43 (probe G, nucleotides 171-506 of pFRK43), revealed further diversity among the tyrosine kinase negative *trk*B transcripts. As shown in FIGURE 2, probe G recognized the tyrosine kinase positive 9.0 kb and 5.5 kb transcripts as well as the tyrosine kinase negative 8.2 kb and 2.5 kb mRNAs. However, the 8.0 kb and 2.0 kb tyrosine kinase negative mRNAs did not hybridize to this probe (FIGURE 2). These results raise the possibility that the *trk*B locus may also code for a third type of receptor-like molecule with different amino-terminal sequences. Alternatively, different promoters may direct the expression of a single protein lacking tyrosine protein kinase sequences (FIGURE 2).

Example 3

Identification of Product Coded for By Tyrosine kinase Negative cDNA Clones

To identify the product coded for by these tyrosine kinase negative *trk*B transcripts, pFRK42 cDNA sequences were subcloned in pMEXneo a mammalian expression vector containing a multiple cloning site flanked by the MSV LTR (Moloney sarcoma virus, long terminal repeat) regulatory sequences and the polyadenylation signal of SV40. See, Martin-Zanca et al., Mol. Cell. Biol. 9: 24-33 (1989). In addition, pMEXneo carries the bacterial neo gene (responsible for resistance to the antibiotic neomycin) driven by the SV40 early promoter. The resulting plasmid, pFRK47, was used to transfect NIH3T3 cells and the resulting G418-resistant colonies submitted to immunoprecipitation analysis.

Transfection of NIH3T3 cells, selection of G418-resistant colonies, metabolic labeling, immunoprecipitation, and SDS-PAGE (sodium dodecyl sulphate-polyacrylamide gel electrophoresis) analysis were done as described in Martin-Zanca, D. et al., Mol. Cell. Biol. 9, 24-33 (1989). Gels were exposed to Kodak X-OMAT film for 3 days with the help of intensifier screens. For Western blot analysis (See, Harlow, E. et al., Antibodies: A Laboratory Manual, Cold Spring Harbor Laboratory, Cold Spring Harbor, NY (1988)), *trk*B-specific proteins were immunoprecipitated from crude brain extracts prior to SDS-PAGE, blotted onto nitro-cellulose filters, incubated again with the respective antisera and visualized using $^{125}$I-protein A (5.6 µCi/µg; 5µCi per 10 ml of Tris-buffered saline) and Kodak X-OMAT film for 6 hours.

Western blotting was performed as follows. Adult Balb/c mouse brain extracts were prepared by homogenizing one gram of brain in 7.5 ml of immunoprecipitation lysis buffer containing 1% Triton X-100, 0.1% sodium dodecyl sulfate, 50 µM NaPO$_4$ pH 7.0, 0.5% Na-deoxycholate and the protease inhibitors aprotinin and phenylmethylsulfonyl fluoride. The lysate was cleared of debris by centrifugation at 3000 rpm for 15 minutes at 4°C followed by high speed centrifugation at 20,000 rpm for 45 minutes at 4°C. 500 µl of the supernatant was incubated overnight at 0°C with 10 µl of antiserum recognizing either the tyrosine kinase domain of tyrosine kinase positive gp145$^{trkB}$ or the carboxy-terminal amino acid residues of tyrosine kinase negative gp95$^{trkB}$ (the glycosylated form of the protein encoded by the pFRK42 tyrosine kinase negative cDNA clone). The antigen-antibody complexes were precipitated by protein A-sepharose beads, washed three times with lysis buffers (without protease inhibitors) and the immunoprecipitated proteins were separated by SDS-PAGE using 8% slab gels. The proteins were then blotted onto nitrocellulose paper using a semi-dry western blotting apparatus (American Biometrics, Inc., Hayward, CA). Before incubating the nitrocellulose with trkB-specific anitsera, the specific binding sites were blocked by incubating the nitrocellulose in Tris-buffered saline (TBS) containing 5% bovine serum albumin and either 1% ovalbumin (in the case of the tyrosine kinase positive-specific antiserum) or 0.2% Tween-20 (in the case of the tyrosine kinase negative-specific antiserum) (all reagents from Sigma Chemical Co., St. Louis, MO). The mitrocellulose was washed four times for 10 minutes in TBS, then incubated for 60 minutes at room temperature with $^{125}$-labeled Protein A (Dupont) (5.6 µCi/µg; 5 µCi per 10 ml of TBS) in blocking solution and again washed four times in TBS. The introcellulose was dried and exposed to KodaK X-OMAT film for 6 hours.

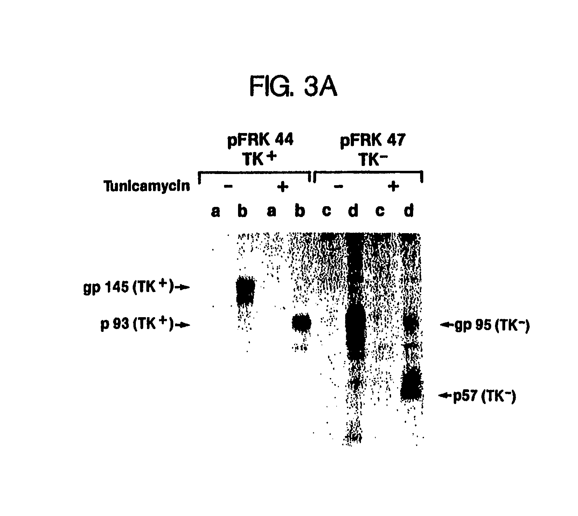

As shown in FIGURE 3A, antibodies raised against the thirteen amino acid residues (positions 464 to 476) located at the carboxy-terminus of the polypeptide predicted to be encoded for by pFRK42 specifically recognized a glycoprotein with an apparent molecular weight of 95,000 daltons (gp95$^{trkB}$) The polypeptide backbone of this molecule was 57,000 daltons as determined by immunoprecipitation of tunicamycin-treated cells. The size of this polypeptide corresponds well with the molecular mass (53,185 daltons) predicted from the deduced amino sequence of the pFRK42 product. This antisera failed to recognize the tyrosine kinase positive gp145$^{trkB}$ molecule. Similarly, antiserum elicited against the carboxy-terminal domain of gp145$^{trkB}$ or against a bacteriasynthesized polypeptide encompassing the entire gp145$^{trkB}$tyrosine kinase domain (residues 547-821) did not precipitate the tyrosine kinase negative gp95$^{trkB}$ glycoprrotein.

The presence of both tyrosine kinase positive gp145$^{trkB}$ and tyrosine kinase negative gp95$^{trkB}$ molecules in brain tissue was next investigated by Western blot analysis. gp145$^{trkB}$ was recognized by the antiserum elicited against a bacteria-synthesized trkB tyrosine protein kinase domain. Similarly, the tyrosine kinase negative gp95$^{trkB}$ molecule could be identified in brain tissue using the antiserum raised against its 13 amine acid carboxy-terminal domain. These results demonstrate that the trkB locus codes for two different neurogenic glycoproteins, which based on their deduced amino acid sequences, possess identical extracellular and transmembrane domains but differ in the presence (gp145$^{trkB}$) or absence (gp95$^{trkB}$) of a tyrosine protein kinase catalytic region. The tyrosine kinase negative gp95$^{trkB}$ protein detected in brain migrated slightly faster in SDS-PAGE than its counterpart expressed in NIH3T3 cells. This observation suggests that glycosylation of gp95$^{trkB}$ in its physiological environment may be different than in fibroblasts.

## Example 4

### In situ Hybridization

The expression in adult mouse brain of the trkB receptors was next investigated. For this purpose, in situ hybridization analysis of a series of coronal sections of 4 week-old mice was performed using three independent probes including (i) a trkB pan probe that identifies all trkB transcripts (both tyrosine kinase positive and tyrosine kinase negative) consisting of nucleotides 670-1152 of pFRK42 which are present in both tyrosine kinase positve and tyrosine kinase negative mRNAs, (ii) a tyrosine kinase positive mRNA specific probe (nucleotides 2767-3057 of pFRK43) and (iii) a tyrosine kinase negative mRNA specific probe (nucleotides 1454-1711 of pFRK42). These probes were prepared by PCR as described in Example 2.

In situ hybridization analysis was essentially performed as described in Hogan, B. L. M. et al., Manipulating the Mouse Embryo, Cold Spring Harbor Laboratory, Cold Spring Harbor, NY (1986) incorporating the modifications described in Klein, R. et al., EMBO J 8, 3701-3709 (1989). Probes D and E were obtained as described in Example 2. These cDNA fragments were subcloned into the pGEM-3Z vector (Promega, Madison, WI) using the procedures described in Maniatis et al., supra. Briefly, the cDNA fragments were digested with the restriction endonuclease Bam HI and ligated with Bam HI-linearized vector plasmid. Transformation of DH5 cells, analysis of miniprep DNA and preparation of large scale plasmid DNA were done as described in Example 1. Nucleotide sequences were determined as described in Example 1 using the T7 primer and modified T7 DNA polymerase (United States Biochemicals). To synthesize [$^{35}$S]-labeled single-stranded antisense cRNA probes, all plasmids were linearized by Hind III digestion and in vitro transcribed using T7 RNA polymerase as described in Klein, R. et al., EMBO J. 8, 3701-3709 (1989).

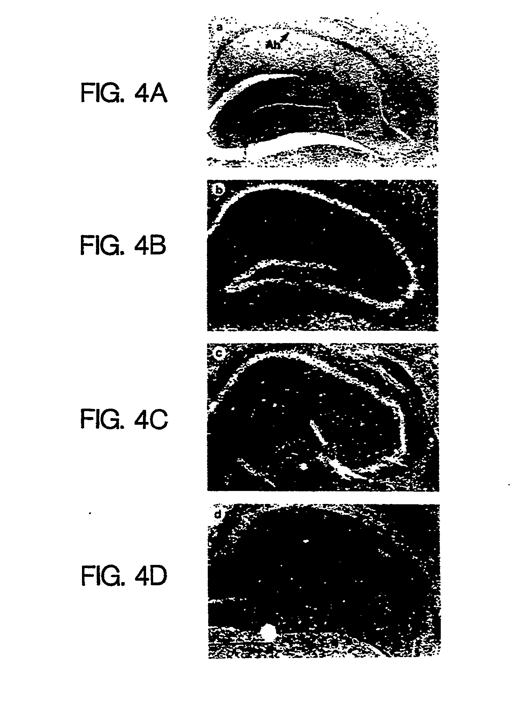

FIGURE 4a shows a section of the brain depicting the Ammon's horn of the hippocampus including the pyramidal cell layer and the dentate gyrus. When this section was hybridized to the trkB pan probe, densely packed cell bodies of those neurons that form the pyramidal cell layer and the dentate gyrus showed high expression of trkB transcripts (FIGURE 4b). In order to ascertain the nature of these transcripts, adjacent sections were hybridized with the tyrosine kinase positive and tyrosine kinase negative mRNA specific probes. As shown in FIGURES 4c and d, only the tyrosine kinase positive probe could recognize specific *trk*B transcripts in this particular section of the adult mouse brain.

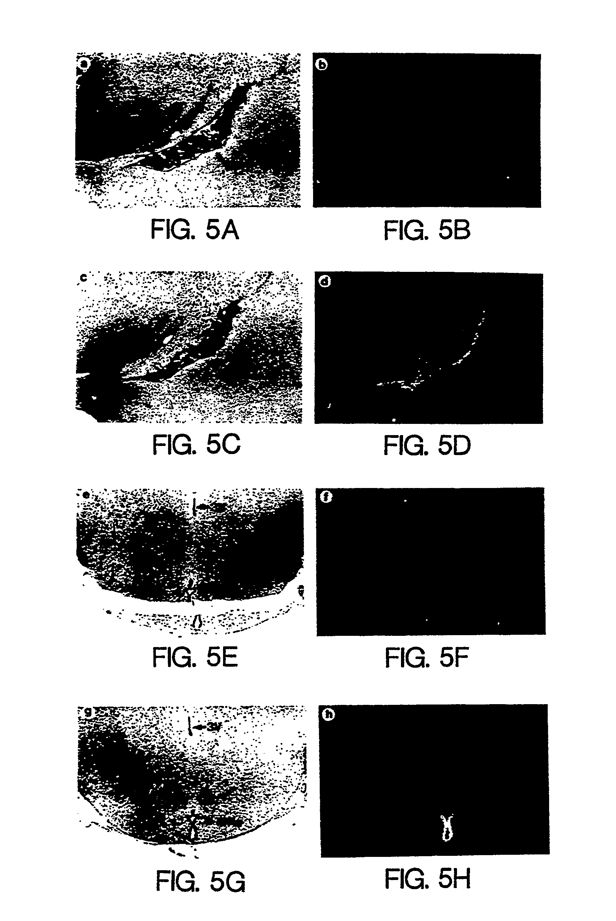

In addition to the hippocampus, the *trk*B pan probe also hybridized to the choroid plexus of the lateral and third ventricles. When these sections were hybridized with the tyrosine kinase positive mRNA specific probe, no expression was observed (FIGURES 5b and 5f). In contrast, the tyrosine kinase negative probe exhibited a strong positive signal (FIGURES 5d and 5h). Interestingly, the tyrosine kinase negative *trk*B transcripts appear to be specifically localized in the choroid plexus and not in adjacent structures since the tissue that outlines the ventricle, the ependyma, did not express any detectable levels of *trk*B transcripts (FIGURE 5H). These results demonstrate that the two classes of *trk*B transcripts (tyrosine kinase positive and tyrosine kinase negative) are differentially expressed within functionally distinct structures of the adult mouse brain.

As illustrated in FIGURE 5, expression of tyrosine kinase negative *trk*B transcripts appear to be restricted (at least in the forebrain) to choroid plexus structures (FIGURE 5). The choroid plexus is known to play a critical role as an active barrier between the blood supply and the cerebral spinal fluid. Therefore, it is possible that the non-catalytic qp95$^{trkB}$ molecule may have a role in the active transport of a putative ligand across the blood/brain barrier. Such a ligand, when brought into the cerebral spinal fluid, may activate the tyrosine kinase positive qp145$^{trkB}$ receptor which is predominantly expressed in neurons of the cerebral cortex and the pyramidal cell layer of the hippocampus.

FIGURE 1. Nucleotide sequence analysis of pFRK42, a tyrosine protein kinase negative cDNA clone of the *trk*B gene. (A) Schematic representation: The thick bar represents coding sequences which are flanked by the putative initiating (ATG) and terminating (TAG) codons. The predicted signal peptide (SP, double hatched box) and transmembrane (TM, solid box) domains are indicated. Those sequences not present in previously characterized *trk* cDNA clones are shaded. Other symbols represent the cysteine residues (closed circles) and consensus N-glycosylation sites (inverted triangles) of the putative ligand-binding domain. (B) Nucleotide and deduced amino acid sequence of the pFRK42 cDNA insert. The putative signal peptide (amino acid residues 1-31) is high-lighted by a shaded bar. Cysteine residues of the putative ligand binding domain (amino acid residues 32-429),are circled. The consensus N-glycosylation sites are underlined by open bars. The putative transmembrane region (amino acid residues 430-453) is underlined by a solid bar. Polyadenylation motifs [AAT(T)AAA] in the 3' noncoding region are underlined. The point of sequence divergence with the tyrosine kinase positive *trk*B cDNA clone pFRK43 is indicated by a vertical arrow.

FIGURE 2. Molecular analysis of *trk*B transcripts in adult mouse tissues. TOP: Northern blot analysis of poly A-containing RNAs isolated from adult mouse brain (Br), liver (Li) and lung (Lu) tissues. [$^{32}$P]-labeled probes include: Probe A (nucleotides 119-490 of pFRK42); probe E: (nucleotides 1454-1711 of pFRK42); probe K (nucleotides 2119-2460 of pFRK42); Probe D (nucleotides 2767-3057 of pFRK43); probe H (nucleotides 2089-2343 of pFRK43); and probe G (nucleotides 171-506 of pFRK43). Migration of the respective *trk*B transcripts is indicated by arrows. Their sizes were calculated from their relative electrophoretic mobility with respect to RNA size standards including S. ceanevisiae 28S (3.4 kb) and 18S (1.8 kb) and *E. coli* 23S (2.9 kb) and 16S (1.5kb) ribosomal RNAs. BOTTOM: Schematic representation of the different *trk*B mRNA species as deduced from the Northern blot analysis. The thick bars represent coding sequences. The thin bars represent 5' and 3' noncoding sequences. The putative signal peptide (SP), transmembrane (TM), and tyrosine protein kianse (TK) domains are indicated by horizontal arrows. Sequences specific to the tyrosine kinase positive (right to left hatching) and tyrosine kinase negative (left to right hatching) *trk*B mRNA species are also indicated. The position and length of the respective probes are indicated by horizontal lines. The vertical arrow indicates the putative polyadenylation sites of the 2.5 kb and 2.0 kb tyrosine kinase negative transcripts.

FIGURE 3. (A) Identification of *trk*B proteins expressed in NIH3T3 cells transfected with tyrosine kinase positive and tyrosine kinase negative *trk*B cDNA clones. LEFT: [$^{35}$S]-methionine-labeled cell extracts of NIH3T3 cells (clone 38-923) transfected with pFRK44, a pMEXneo derived expression plasmid carrying the tyrosine kinase positive cDNA insert of pFRK43 (See, Klein, R. et al., EMBO J. 8, 3701-3709 (1989)) were immunoprecipitated with a rabbit antiserum raised against a peptide corresponding to carboxy-terminal sequences (amino acid residues 794-808) of the tyrosine kinase positive *trk*B receptor-like protein (See, Klein, R. et al., EMBO J. 8, 3701-3709 (1989)) either (a) in the presence or (b) in the absence of 10μg of the peptide (NH$_2$ - KGFVLFHKIPLDGCOOH) used to generate the antiserum. RIGHT: [$^{35}$S]-methionine-labeled cell extracts of NIH3T3 cells (clone B24-76) transfected with pFRK47, a pMEXneo derivative carrying the tyrosine kinase negative *trk*B cDNA insert of pFRK42, were immunoprecipitated with a rabbit antiserum raised against a peptide corresponding to the 13 carboxy-terminal amino acid residues of the putative tyrosine kinase negative *trk*B protein (FIGURE 1) either (c) the presence of or (d) in the absence of 10 μg of competing peptide. Parallel cultures were metabolically labeled (-) in the absence of or (+) in the presence of 10 μg/ml of tunicamycin. The migration of the glycosylated (gp145 and gp97) and unglycosylated (p93 and p57) *trk*B products is indicated by arrows. The molecular weights of these *trk*B proteins was deduced from their electrophoretic migration relative to molecular weight standards including myosin (200,000), phosphorylase B (92,500), albumin (69,000) and ovalbumin (43,000).

FIGURE 4. trkB expression in the hippocampal region of adult mouse brain. (A) light-field view of a coronal section showing the Ammon's horn (Ah) and dentate gyrus (DG). Dark-field view of (b) the section shown in panel a and of (c,d) adjacent sections after in situ hybridization to trkB-specific antisense cRNA probes including (b) a pan-probe (nucleotides 670-1152 of pFRK42), (c) tyrosine kinase positive-specific probe D (nucleotides 2767-3057 of pFRK43) and (d) tyrosine kinase negative-specific probe E (nucleotides 1454-1711 of pFRK42).

FIGURE 5. trkB expression in the choroid plexus of adult mouse brain. Panels a, c, e and g are light-field views of coronal sections depicting the lateral ventricle (LV), the 3rd ventricle (3V), and the mammillary recess of the 3rd ventricle (MR3). Panels b, d, f and h show the correspondign dark-field views after in situ hybridization to the tyrosine kinase positive-specific probe D (panels b and f) and to the tyrosine kinase negative-specific probe E (panels d and h) as described above.

## Claims

1. An isolated nucleic acid molecule comprising a nucleic acid sequence coding for all or part of a tyrosine kinase negative trkB protein.

2. The nucleic acid molecule according to Claim 1 which is a DNA molecule and wherein the nucleic acid sequence is a DNA sequence.

3. The DNA molecule according to Claim 2 wherein the DNA sequence has the nucleotide sequence substantially as shown in Figure 1B.

4. The DNA molecule according to Claim 2 wherein the DNA sequence has part of the nucleotide sequence substantially as shown in Figure 1B.

5. The DNA molecule according to Claim 4 wherein the DNA sequence has a nucleotide sequence corresponding to nucleotides 1 to 1429 as shown in Figure 1B.

6. A DNA molecule having a DNA sequence which is complementary to the DNA sequence according to any one of Claims 3, 4, or 5.

7. An expression vector containing a DNA sequence coding for all or part of a tyrosine kinase negative trkB protein.

8. The expression vector according to Claim 7 comprising one or more control DNA sequences capable of directing the replication and/or the expression of and operatively linked to the DNA sequence coding for all or part of a tyrosine kinase negative trkB protein.

9. The expression vector according to Claim 7 wherein the DNA sequence coding for all or part of a tyrosine kinase negative trkB protein has the nucleotide sequence substantially as shown in Figure 1B.

10. The expression vector according to Claim 7 wherein the DNA sequence coding for all or part of a tyrosine kinase negative trkB protein has part of the nucleotide sequence substantially as shown in Figure 1B.

11. The expression vector according to Claim 9 designated pFRK42.

12. An expression vector having the identifying characteristics of the expression vector according to Claim 11.

13. A prokaryotic or eukaryotic host cell transformed or transfected with the expression vector according to any one of Claims 7 to 12.

14. The host cell according to Claim 13 wherein the host cell is a mammalian cell.

15. The host cell according to Claim 14 wherein the mammalian cell is an NIH3T3 cell.

16. A method for producing a polypeptide molecule which comprises all or part of a tyrosine kinase negative *trk*B protein comprising culturing a host cell according to Claim 13 under conditions permitting expression of the polypeptide.

17. A method for detecting a nucleic acid sequence coding for all or part of a tyrosine kinase negative *trk*B protein comprising contacting the nucleic acid sequence with a detectable marker which binds specifically to at least part of the nucleic acid sequence, and detecting the marker so bound, the presence of bound marker indicating the presence of the nucleic acid sequence.

18. The method according to Claim 17 wherein the nucleic acid sequence is a DNA sequence.

19. The method according to Claim 17 wherein the nucleic acid sequence is an RNA sequence.

20. The method according to Claim 18 wherein the DNA sequence has the nucleotide sequence substantially as shown in Figure 1B.

21. The method according to Claim 18 wherein the DNA sequence has part of the nucleotide sequence substantially as shown in Figure 1B.

22. The method according to any one of Claims 17 to 21 wherein the detectable marker is a nucleotide sequence complementary to at least a portion of the nucleic acid sequence.

23. The method acording to Claim 22 wherein the nucleotide sequence of the detectable marker is selected from a cDNA sequence, an RNA sequence, a sense RNA sequence or an antisense RNA sequence.

24. The method according to any one of Claims 18 to 23 wherein the detectable marker is labelled with a radioisotope.

25. The method according to Claim 24 wherein the detecting is by autoradiography.

26. The method according to Claim 18 wherein the DNA sequence is a genomic DNA sequence.

27. The method according to Claim 19 wherein the RNA sequence is a messenger RNA sequence.

28. The method according to Claim 19 wherein the RNA sequence is located in the cells of a tissue sample.

29. An isolated polypeptide molecule comprising all or part of a tyrosine kinase negative *trk*B protein.

30. An isolated polypeptide molecule encoded by the DNA sequence according to Claim 2

31. The polypeptide molecule according to Claim 29 having the amino acid sequence substantially as shown in Figure 1B.

32. The polypeptide molecule according to Claim 29 having part of the amino acid sequence substantially as shown in Figure 1B.

33. A polypeptide molecule having an amino acid sequence which is at least about 90% homologous to the amino acid sequence according to Claims 31 or 32.

34. The polypeptide molecule according to Claim 29 which is glycosylated.

# FIG. 1A

AMINO ACIDS
RESIDUES

kbp

```
   1 ATGTCGCCCTGGCTGAAGTGGCATGGACCCGCCATGGCGCGGCTCTGGGGGCTTATGCCTG  60
     [M  S  P  W  L  K  W  H  G  P  A  M  A  R  L  W  G  L  C  L   20

  61 CTGGTCTTGGGCTTCTGGAGGGCCTCTCTCGCCTGCCCGACGTCCTGCAAATGCAGTTCC  120
     L  V  L  G  F  W  R  A  S  L  A] ©  P  T  S  ©  K  ©  S  S    40

 121 GCTAGGATTTGGTGTACTGAGCCTTCTCCAGGCATCGTGGCATTCCCGAGGTTGGAACCT  180
     A  R  I  W  ©  T  E  P  S  P  G  I  V  A  F  P  R  L  E  P    60

 181 AACAGCGTTGACCCGGAGAACATCACGGAAATTCTCATTGCAAACCAGAAAAGGCTAGAA  240
     N  S  V  D  P  E  N  I  T  F  I  L  I  A  N  Q  K  R  L  E    80

 241 ATCATCAATGAAGATGACGTTGAAGCTTACGTGGGGGCTGAGAAACCTTACAATTGTGGAT  300
     I  I  N  E  D  D  V  E  A  Y  V  G  L  R  N  L  T  I  V  D   100

 301 TCCGGCTTAAAGTTTGTGGCTTACAAAGCGTTTCTGAAAAACAGCAACCTGCGGCACATA  360
     S  G  L  K  F  V  A  Y  K  A  F  L  K  N  S  N  L  R  H  I   120

 361 AATTTCACACGAAACAAGCTGACGAGTTTGTCCAGGAGACATTTCCGCCACCTTGACTTG  420
     N  F  T  R  N  K  L  T  S  L  S  R  R  H  F  R  H  L  D  L   140

 421 TCTGACCTGATCCTGACGGGTAATCCGTTCACGTGCTCCTGCGACATCATGTGGCTCAAG  480
     S  D  L  I  L  T  G  N  P  F  T  ©  S  ©  D  I  M  W  L  K   160

 481 ACTCTCCAGGAGACTAAATCCAGCCCCGACACTCAGGATTTGTACTGCCTCAATGAGAGC  540
     T  L  Q  E  T  K  S  S  P  D  T  Q  D  L  Y  ©  L  N  E  S   180

 541 AGCAAGAACATGCCCCTGGCGAACCTGCAGATACCCAATTGTGGTCTGCCATCTGCACGT  600
     S  K  N  M  P  L  A  N  L  Q  I  P  N  ©  G  L  P  S  A  R   200

 601 CTGGCTGCTCCTAACCTCACCGTGGAGGAAGGAAAGTCTGTGACCCTTTCCTGCAGTGTG  660
     L  A  A  P  N  L  T  V  E  E  G  K  S  V  T  L  S  ©  S  V   220

 661 GGGGGTGACCCACTCCCCACCTTGTACTGGGACGTTGGGAATTTGGTTTCCAAGCACATG  720
     G  G  D  P  L  P  T  L  Y  W  D  V  G  N  L  V  S  K  H  M   240

 721 AATGAAACAAGCCACACACAGGGCTCCTTAAGGATAACGAACATTTCATCTGATGACAGT  780
     N  E  T  S  H  T  Q  G  S  L  R  I  T  N  I  S  S  D  D  S   260

 781 GGAAAGCAAATCTCTTGTGTGGCAGAAAACCTTGTAGGAGAAGATCAAGATTCTGTGAAC  840
     G  K  Q  I  S  ©  V  A  E  N  L  V  G  E  D  Q  D  S  V  N   280

 841 CTCACTGTGCATTTTGCGCCAACTATCACGTTTCTCGAGTCTCCAACCTCAGATCACCAC  900
     L  T  V  H  F  A  P  T  I  T  F  L  E  S  P  T  S  D  H  H   300

 901 TGGTGCATTCCATTCACTGTGAGAGGCAACCCCAAGCCTGCGCTTCAGTGGTTCTACAAT  960
     W  ©  I  P  F  T  V  R  G  N  P  K  P  A  L  Q  W  F  Y  N   320

 961 GGGGCCATACTGAATGAGTCCAAGTACATCTGTACTAAGATCCACGTCACCAATCACACG 1020
     G  A  I  L  N  E  S  K  Y  I  ©  T  K  I  H  V  T  N  H  T   340

1021 GAGTACCATGGCTGCCTCCAGCTGGATAACCCCACTCATATGAATAACGGAGACTACACC 1080
     E  Y  H  G  ©  L  Q  L  D  N  P  T  H  M  N  N  G  D  Y  T   360
```

## FIG. 1B-1

```
1081 CTGATGGCCAAGAACGAGTATGGGAAGGATGAGAGACAGATCTCCGCTCACTTCATGGGC 1140
       L  M  A  K  N  E  Y  G  K  D  E  R  Q  I  S  A  H  F  M  G  380

1141 CGGCCTGGAGTCGACTACGAGACAAACCCAAATTACCCTGAAGTCCTCTATGAAGACTGG 1200
       R  P  G  V  D  Y  E  T  N  P  N  Y  P  E  V  L  Y  E  D  W  400

1201 ACCACGCCAACTGACATTGGGGATACTACGAACAAAAGTAATGAAATCCCCTCCACGGAT 1260
       T  T  P  T  D  I  G  D  T  T  N  K  S  N  E  I  P  S  T  D  420

1261 GTTGCTGACCAAAGCAATCGGGAGCATCTCTCGGTCTATGCCGTGGTGGTGATTGCATCT 1320
       V  A  D  Q  S  N  R  E  H  L  S  V  Y  A  V  V  V  I  A  S  440

1321 GTGGTGGGATTCTGCCTGCTGGTGATGTTGCTCCTGCTCAAGTTGGCGAGACATTCCAAG 1380
       V  V  G  F  C  L  L  V  M  L  L  L  K  L  A  R  H  S  K  460

1381 TTTGGCATGAAAGGTTTTGTTTTGTTTCATAAGATCCCACTGGATGGGTAGCTGAGATAA 1440
       F  G  M  K  G  F  V  L  F  H  K  I  P  L  D  G  ***  476

1441 AGGAAAGACAAAGGCTGGGGCTGTGGTGCTTGTTGCCTGACGCCCTGTGAGCTGAACTCT 1500

1501 GGGACTGCTGTTGCCTATCCCAGGAAGTGCTGCTTATTTGAGGGTGTCTGGTGGAAATGG 1560

1561 GTAATCTCCGAGGATGTCTGCAGCCTGCTTGTTGTGAGCTGTGACTGGGGAACCCCAAGG 1620

1621 CAGAGGCAGGGGTCAGGCAGCTGAGAAGCAGCAGAAGAACACACTTAGATTCACCTTCTG 1680

1681 TTCTTACAATAGTTCAAATATAGAATCGAAGTGAAATCTCATTGGATTATGCCTCTCTAA 1740

1741 TGAAAAGCGAGCTGTTTGACTATACGGAAAATGTGCTGACATTAATTGCTTCTGTTTATT 1800

1801 AAAGGTGATTTGCAAATTAAAAACTCTGCATCTATCATCTATCCATCTATCTGTTTGTCT 1860

1861 ATCATATCTATCTGTCTGTCTATCTGTCTATCATCTATCTACCTACCTCTCTATCATATC 1920

1921 TATCTGTCTGTCTATCTATCTATCTATCTATCTATCTATCTATCTATCTATCTATCTATC 1980

1981 TATCTATCATCTATCTACCTATCATCGATCTACTTATCTATCATCTATCTATCTACCTAT 2040

2041 CATCGATTTACTTATCTATCATCTATCTATCTATCTATCTATCTATCTATCTATCTATCT 2100

2101 ATCTGTCATCTATCTAAAGTCATAGCTAGGTCTAAGTGCACACTAAAAGTCTAATCCACA 2160

2161 CATAACACCTATTTCAGCAACATCTTCTGTTCTCTAACCTTTGCTAACTTCTGTGATTTC 2220

2221 CACCTACAACCCTGCGACTGATAGACTTAAAGGCACATTGGTGGTGTCATTAGTAGGTTC 2280

2281 TTTGTTTTGCTGGCAGCAAAGACCCAAACTCTTCGCTAACGATTGCTTTCAAAGTCCACC 2340

2341 CGGCAGGTAGAACGGAGCAGCACCAGGGACTGTGTGGCCAGGAGTATGGACCTGAATTAA 2400

2401 CCACAGCCTGAGAATAAATAATGGTAGGGTATATGCATATAGGGAATTAAAATCTTGTCC 2460

2461 CTTTCCATTGCCCTCTGCTAACCG 2484
```

## FIG. 1B-2

FIG. 2

Probe A E K D H G

Br Li Lu  Br Li Lu  Br Li Lu  Br Li Lu  Br Li Lu  Br Li Lu

kb                                                           kb

9.0 →                                                        ← 9.0
8.2 →                                                        ← 8.2
8.0 →                                                        ← 8.0

5.5 →                                                        ← 5.5

2.5 →                                                        ← 2.5
2.0 →                                                        ← 2.0

mRNA species

SP          TM        TK
G   A              H        D          $A_n$   9.0kb, 5.5kb

SP          TM
G   A              E        K          $A_n$   8.2kb, 2.5kb

TM
?   A              E        K          $A_n$   8.0kb, 2.0kb

500b

EP 0 455 460 A2

# FIG. 3A

pFRK 44
TK⁺     pFRK 47
TK⁻

Tunicamycin    −        +        −        +

a    b    a    b    c    d    c    d

gp 145 (TK⁺) →

p 93 (TK⁺) →                              ← gp 95 (TK⁻)

← p57 (TK⁻)

FIG. 4A

FIG. 4B

FIG. 4C

FIG. 4D

FIG. 5A

FIG. 5B

FIG. 5C

FIG. 5D

FIG. 5E

FIG. 5F

FIG. 5G

FIG. 5H